## Europäisches Patentamt

## European Patent Office

## Office européen des brevets

(19)

(11) Veröffentlichungsnummer: **0 226 732**
**B1**

(12) **EUROPÄISCHE PATENTSCHRIFT**

(45) Veröffentlichungstag der Patentschrift:
03.01.90

(51) Int. Cl.⁴: **G03B 15/00**

(21) Anmeldenummer: 86113822.0

(22) Anmeldetag: 06.10.86

(54) Vorrichtung zum fotogrammetrischen Erfassen des menschlichen Kopfes.

(30) Priorität: 23.12.85 DE 3545875

(43) Veröffentlichungstag der Anmeldung:
01.07.87 Patentblatt 87/27

(45) Bekanntmachung des Hinweises auf die Patenterteilung:
03.01.90 Patentblatt 90/1

(84) Benannte Vertragsstaaten:
AT BE CH DE ES FR GB GR IT LI LU NL SE

(56) Entgegenhaltungen:
DE-A- 3 518 215
US-A- 2 339 657
US-A- 2 780 004

PROCEEDINGS OF THE SOCIETY OF PHOTO-OPTICAL
INSTRUMENTATION ENGINEERS, SPIE, Band 166, 1978,
Seiten 235-243; G. AKERSKOG et al.: "Children's
glasses"

(73) Patentinhaber: EYEMETRICS-SYSTEMS AG,
Steinbockstrasse 4, CH-7001 Chur(CH)

(72) Erfinder: Steinhauer, Eric, 2238 Richey Drive, La Canada,
Ca. 91911(US)
Erfinder: Lange, Karl-Heinz, Dobergstrasse 112,
D-4980 Bünde 18(DE)

(74) Vertreter: Grams, Klaus Dieter, Dipl.-Ing. et al,
Patentanwaltsbüro Tiedtke-Bühling-Kinne-
Grupe-Pellmann-Grams-Struif Winter-Roth
Bavariaring 4, D-8000 München 2(DE)

**Beschreibung**

Die Erfindung bezieht sich auf eine Vorrichtung zum fotogrammetrischen Erfassen des menschlichen Kopfes gemäß dem Oberbegriff von Patentanspruch 1.

Eine Vorrichtung mit den Merkmalen des Oberbegriffs von Patentanspruch 1 ist bekannt (SPIE Vol. 166 Applications of Human Biostereometrices — (NATO) (1978), Seiten 235 bis 243). Mittels dieser bekannten Vorrichtung kann die Vorderseite des Kopfes eines Menschen, d.h. im wesentlichen sein Gesicht, seine Schläfenbereiche und die Ohren, fotografisch aufgenommen werden, während ein geeignetes Musterbild auf das Gesicht projiziert wird. Dabei werden gleichzeitig zwei Aufnahmen aus verschiedenen Richtungen gemacht, deren Auswertung es ermöglicht, die Geometrie des aufgenommenen Berichts des Kopfes dreidimensional zu erfassen. Die bekannte Vorrichtung dient dazu, durchschnittliche Verläufe von Nasenprofilen und durchschnittliche Abstände wie beispielsweise durchschnittliche Augenabstände, durchschnittliche Abstände der Nasenwurzel von der Ohrwurzel in Seitenansicht, durchschnittliche Ohrwurzelabstände und dergleichen zu messen, um aufgrund dieser Daten die Abmessungen von Brillenfassungen festzulegen.

Wenig Information kann mittels der bekannten Vorrichtung über die Bereiche zwischen den beiden Ohren und dem Schädel des Kopfes gewonnen werden. Solche Informationen bzw. Daten sind insbesondere dann erwünscht, wenn aufgrund der fotogrammetrischen Messung individuelle, genaue Daten für die Anfertigung einer Brille gewonnen werden sollen.

Der Erfindung liegt die Aufgabe zugrunde, die gattungsgemäße Vorrichtung derart auszubilden, daß sie fotogrammetrische Messungen mit möglichst geringen Anstrengungen für die Person, deren Kopf ausgemessen werden soll, und die Bedienperson der Vorrichtung ermöglicht und dass zugleich mit der Ausmessung des Gesichtes die Bereiche zwischen den beiden Ohren und dem Schädel des Kopfes hinreichend durch Messung erfaßbar sind.

Diese Aufgabe wird erfindungsgemäß gelöst durch die Merkmale im kennzeichnenden Teil von Patentanspruch 1.

Bei der erfindungsgemäßen Vorrichtung ist ein Geräteträger vorgesehen, der sowohl die Kameraeinrichtung als auch den Projektor trägt und zusammen mit diesen eine im folgenden auch als optischer Kopf bezeichnete Einheit bildet. Dieser optische Kopf und der menschliche Kopf sind relativ zueinander in senkrechter Richtung und außerdem zumindest in einer Richtung horizontal bewegbar, wobei diese eine Richtung im wesentlichen mit der optischen Achse der Kameraeinrichtung zusammenfällt.

Am Geräteträger oder der Sitzgelegenheit für die Person, deren Kopf fotogrammetrisch erfaßt werden soll, sind mit Hilfe von Parallelführungseinrichtungen zwei Ohrtaster angebracht, die jeweils an den Bereich zwischen einem der Ohren und dem Schädel des Kopfes anlegbar sind. Diese Ohrtaster weisen jeweils Marken auf, die bei angelegtem Ohrtaster zugleich mit dem Gesicht bzw. der Vorderseite des Kopfes sichtbar sind, so daß durch die fotogrammetrische Erfassung dieser Marken zugleich mit den Daten, die beispielsweise den Bereich der Nase, der Augenhöhlen, der Brauen, der Jochbeine und der Schläfen beschreiben, Daten über den Bereich zwischen Ohr und Schädel des Kopfes gewonnen werden.

Durch die US-A 2 339 657 ist eine Vorrichtung zum Fotografieren des menschlichen Kopfes bekannt, bei der eine Kameraeinrichtung und eine Beleuchtungseinrichtung von einem Geräteträger getragen werden, der relativ zu einem vorrichtungsfesten Unterbau in senkrechter Richtung und in einer Horizontalrichtung bewegbar ist.

Voraussetzung für eine genaue fotogrammetrische Messung der vorstehend beschriebenen Art ist, daß sich das Objekt, d.h. die Vorderseite des Kopfes, relativ zur Kameraeinrichtung und zum Projektor in einer bestimmten, bekannten Objektebene und somit Objektentfernung befindet. Im bekannten Fall wird dies dadurch erreicht, daß die Kameraeinrichtung und der Projektor auf die Objektebene ausgerichtet und die Objektentfernung eingestellt werden und daß die Person, deren Kopf vermessen werden soll, so ausgerichtet werden muß, daß ihr Gesicht sich möglichst genau in der Objektebene befindet. Dieser Vorgang erfordert Kooperationsbereitschaft von Seiten der Person und außerdem verhältnismäßig viel Zeit. Darüber hinaus ist festgestellt worden, daß durch das Ausrichten des Kopfes bisweilen eine angespannte und unnatürliche Haltung eingenommen wird, die sich wiederum im Ausdruck des Gesichtes wiederspiegelt und möglicherweise zu Verzerrungen des Gesichtes führt, die einer genauen Messung entgegenstehen.

Um einerseits eine schnelle Durchführbarkeit der Messung und andererseits einen entspannten, natürlichen Gesichtsausdruck zu ermöglichen, kann in vorteilhafter Ausbildung der Erfindung vorgesehen sein, daß die Relativbewegbarkeit zwischen dem menschlichen Kopf und dem Geräteträger dadurch erreicht wird, daß der Geräteträger in der senkrechten Richtung und der einen Horizontalrichtung relativ zum vorrichtungsfesten Unterbau verfahrbar ist. Diese Ausbildung ermöglicht es, bei der Fotogrammetrie in der Weise vorzugehen, daß die Person, deren Kopf ausgemessen werden soll, auf einer Sitzgelegenheit Platz nimmt und dann eine bequeme Sitzhaltung mit im wesentlichen aufrechtem Oberkörper und gerader Kopfhaltung einnimmt. Bei dieser Positionierung der Person braucht nicht darauf geachtet zu werden, daß ihr Gesicht in einer bestimmten Ebene liegt; vielmehr soll und kann die Person eine bequeme und entspannte Haltung einnehmen, wobei sich die Ebene und der Ort im Raum, in bzw. an dem sich der vordere Bereich des Kopfes der Person befindet, nach den individuellen Eigenschaften der Person richten. Nachdem die Person die vorstehend beschriebene Haltung eingenommen hat, wird dann der optische Kopf aufgrund der erfindungsgemäß gegebenen Bewegbarkeit in eine solche Höhe und in einen solchen Abstand vom Gesicht gefahren, daß der vordere Bereich des Kopfes bzw. das Gesicht sich in der an der Kameraeinrichtung und dem Projektor eingestellten Objektebene und dem Sehfeld der Kameraeinrichtung befindet. Wenn der optische Kopf diese Position erreicht hat, kann die zumindest eine fotogrammetrische Aufnahme ausgeführt werden. Von der Person, deren Kopf vermessen werden soll, wird dabei nicht mehr verlangt, als daß sie sich bequem aufrecht hinsetzt. Die Bedienperson braucht den optischen Kopf lediglich in genannter Weise zu verfahren und weder selbst das Gesicht manuell auszurichten, noch die Person durch Anweisungen dazu zu bringen, ihren Kopf in eine bestimmte, vorgegebene Position zu bewegen.

Die Kameraeinrichtung ist vorzugsweise in der Lage, gleichzeitig aus zwei verschiedenen Richtungen zwei Aufnahmen zu machen, was beispielsweise mit zwei Kameras möglich ist, deren Sehfelder sich zumindest überlappen. Jede dieser beiden Kameras hat eine eigene optische Achse; als optische Achse der Kameraeinrichtung kann in diesem Fall beispielsweise eine in der Mitte zwischen den optischen Achsen der beiden Kameras verlaufende Achse angesehen werden. Die für die erfindungsgemäße Vorrichtung wesentliche Bewegbarkeit in der einen Horizontalrichtung ist gegeben, wenn der optische Kopf der Vorrichtung zum zu messenden menschlichen Kopf und von diesem weg bewegbar ist, während sich der menschliche Kopf im Sehfeld der Kameraeinrichtung befindet. Die Bewegbarkeit in der einen Horizontalrichtung ist auch dann gegeben, wenn der Bewegung in der Horizontalrichtung eine Querkomponente überlagert ist, d.h. gleichzeitig beispielsweise eine Abwärts- oder Aufwärtsbewegung stattfindet. Entsprechendes galt für die Bewegung des optischen Kopfes in senkrechter Richtung; diese ist auch dann gegeben, wenn der Bewegung in senkrechter Richtung gleichzeitig eine Querkomponente überlagert ist, d.h. beispielsweise gleichzeitig eine Bewegung nach vorn (zum menschlichen Kopf) oder nach hinten erfolgt.

In vorteilhafter Ausbildung der Erfindung kann vorgesehen sein, daß der Geräteträger in einer zweiten Horizontalrichtung bewegbar ist, die im wesentlichen senkrecht zur ersten Horizontalrichtung verläuft. Dies erleichtert es, durch Bewegen des optischen Kopfes das Gesicht auch dann in das vorgegebene Sehfeld und die vorgegebene Objektebene der Kameraeinrichtung zu bringen, wenn die Person deutlich seitlich geneigt auf der Sitzgelegenheit sitzt.

In weiterer vorteilhafter Ausbildung der Erfindung sind elektromotorische Antriebseinrichtungen für die Bewegungen des Geräteträgers bzw. optischen Kopfes in den ihm möglichen Bewegungsrichtungen vorgesehen.

Zusätzlich zur vorstehend erläuterten Bewegbarkeit des Geräteträgers bzw. optischen Kopfes kann vorgesehen sein, daß das vom Projektor projizierte Musterbild bei ruhendem optischen Kopf feineinstellbar ist, was durch Schwenken des Projektors und Drehen eines Diapositivs im Projektor erreicht sein kann, das die Vorlage für das Musterbild trägt. Durch diese Ausbildung ist es auf einfache Weise möglich, das Musterbild in eine bestimmte räumliche Zuordnung zu bestimmten Merkmalen des Gesichts zu bringen, das die Auswertung der beiden Aufnahmen erleichtert.

In weiterer vorteilhafter Ausbildung der Vorrichtung kann vorgesehen sein, daß am Geräteträger eine Haube mit zwei Seitenwänden, einer Bodenwand, einer oberen Wand und einer Frontwand befestigt ist, wobei die Frontwand mit einer Öffnung für den Kopf versehen ist und vom Geräteträger annähernd denjenigen Abstand hat, den während der Messung der Kopf vom Geräteträger hat, und wobei die Haube den Raum zwischen der Kameraeinrichtung und dem Projektor einerseits sowie der Frontwand andererseits abdunkelt. Durch eine solche Haube ist der Beeinflussung der Messungen durch Störlicht vorgebeugt. Darüber hinaus sind in der Haube vorzugsweise eine Entfernungsmeßeinrichtung, eine Augenhöhlenbeleuchtungseinrichtung und ein Kalibrierobjekt angeordnet. Die Entfernungsmeßeinrichtung, die vorzugsweise durch zwei Lichtschranken gebildet ist, mißt den Zustand, daß der zu messende Kopf den Sollabstand von der Kameraeinrichtung hat, d.h. sich in der vorgegebenen, bestimmten Objektebene befindet, auf die die Kameraeinrichtung und der Projektor eingestellt sind. Die Augenhöhlenbeleuchtungseinrichtung hat sich als zweckmäßig erwiesen, um einer zu starken Abschattung der Augenhöhlen und einer dadurch bedingten Kontrastarmut der Aufnahmen im Bereich der Augenhöhlen vorzubeugen. Von besonderem Vorteil ist schließlich das Kalibrierobjekt, das vorzugsweise zwischen einer Ruhestellung und einer Kalibrierstellung bewegbar ist und durch die Anordnung in der Haube mittelbar fest mit dem Geräteträger bzw. dem optischen Kopf verbunden ist. Dies gewährleistet einerseits, daß das Kalibrierobjekt im Bedarfsfall jederzeit zu Kalibrierzwecken zur Verfügung steht und andererseits in seiner Kalibrierstellung eine in hohem Maße reproduzierbare Position bezüglich der Kameraeinrichtung einnimmt.

Weitere vorteilhafte Ausgestaltungen der Erfindung ergeben sich aus den Unteransprüchen sowie der folgenden Beschreibung von Ausführungsbeispielen unter Bezugnahme auf die Zeichnungen. Es zeigen:

Fig. 1 eine Seitenansicht eines ersten Ausführungsbeispiels der Vorrichtung;
Fig. 2 eine perspektivische Ansicht wesentlicher Teile der Vorrichtung gemäß Fig. 1;
Fig. 3 in Fig. 2 ähnliche Ansicht eine zweite Ausführungsform der Vorrichtung;
Fig. 4 eine Draufsicht auf einen Schlitten der Ausführungsform gemäß Fig. 3;
Fig. 5 eine Seitenansicht des Schlittens gemäß Fig. 4 von links in Fig. 4;
Fig. 6 in vergrößertem Maßstab einen Schnitt gemäß A-B in Fig. 8 durch einen optischen Kopf und eine fest mit diesem verbundene Kammereinheit der Ausführungsform gemäß den Fig. 1 und 2;
Fig. 7 eine Schnittdarstellung gemäß C-D in Fig. 6;
Fig. 8 eine verkleinerte Draufsicht zu Fig. 6, wobei obere Wände der Kammereinheit und des optischen Kopfes gemäß Fig. 6 aggenommen sind;

Fig. 9 eine Vorderansicht eines menschlichen Kopfes während einer Messung bei Betrachtung entlang der optischen Achse eines Projektors der Vorrichtung;

Fig. 10 eine Draufsicht auf wesentliche Teile einer dritten Ausdfführungsform der Vorrichtung;

Fig. 11 eine Seitenansicht der Ausführungsform gemäß Fig 10; und

Fig. 12 eine Seitenansicht der dritten Ausführungsform der Vorrichtung von rechts in Fig. 11.

In den Figuren bezeichnen gleiche Bezugszeichen gleiche bzw. entsprechende Teile und Elemente.

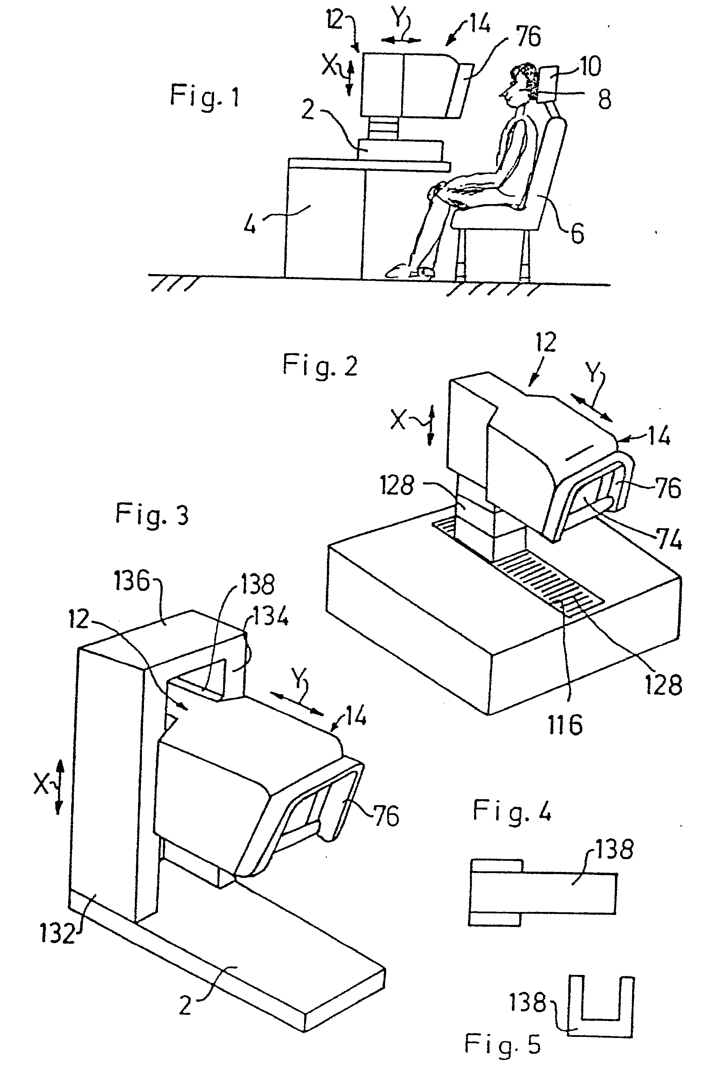

Die in den Fig. 1, 2, 6, 7 und 8 dargestellte erste Ausführungsform der Vorrichtung umfaßt einen kastenförmigen Unterbau 2, der auf einem Tisch 4 steht. Vor dem Unterbau 2 und dem Tisch 4 ist eine Sitzgelegenheit 6 in Form eines hochlehnigen Stuhles angeordnet, auf dem die Person Platz nehmen kann, deren Kopf 8 fotogrammetrisch erfaßt werden soll. Die Sitzgelegenheit 6 trägt am oberen Ende ihrer Rückenlehne eine einstellbare Kopfstütze 10.

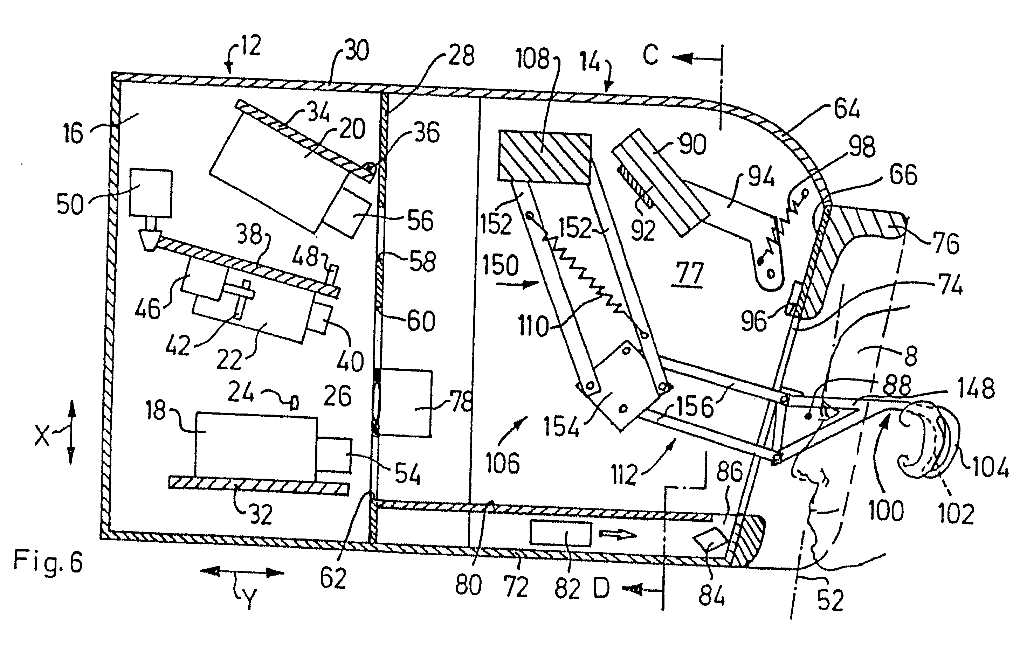

Oberhalb des vorrichtungsfesten Unterbaus 2 sind ein optischer Kopf 12 sowie eine Kammereinheit 14 angeordnet. Der optische Kopf 12 und die Kammereinheit 14 sind fest miteinander verbunden und gemeinsam relativ zum Unterbau 2 in senkrechter Richtung und in einer Horizontalrichtung bewegbar, wie dies durch den Doppelpfeil X für die senkrechte Richtung und durch den Doppelpfeil Y für die Horizontalrichtung in den Fig. 1 und 2 angedeutet ist. Im folgenden werden zunächst unter Bezugnahme auf die Fig. 6 bis 8 der optische Kopf 12 und die Kammereinheit 14 ausführlicher erläutert.

Der optische Kopf 12 umfaßt mehrere optische Geräte sowie einen Geräteträger 16, der die optischen Geräte trägt. Bei den optischen Geräten handelt es sich um eine untere, als Videokamera ausgebildete Kamera 18, eine obere, als Videokamera ausgebildete Kamera 20, einen Projektor 22, ein Beobachtungsobjekt 24 und eine diesem zugeordnete Linsenanordnung 26. Der Geräteträger 16 umfaßt beim dargestellten Ausführungsbeispiel ein selbsttragendes, steifes Gehäuse mit einer in Fig. 6 rechten Vorderwand 28, einer oberen Wand 30 sowie weiteren, in den Figuren nicht bezeichneten Wänden und mit Halterungen für die optischen Geräte. Bei diesen Halterungen handelt es sich um eine erste Halterung 32, die auf nicht dargestellte Weise mit dem übrigen Geräteträger 16 verbunden ist und an der die untere Kamera 18 lösbar und austauschbar befestigt ist. Ferner gehört zu den Halterungen eine zweite Halterung 34, an der die obere Kamera 20 lösbar und austauschbar befestigt ist. Die zweite Halterung 34 ist am übrigen Geräteträger 16 derart angebracht, daß diese Halterung und somit die von ihr getragene obere Kamera 20 sowohl in der Zeichenebene von Fig. 6 um eine Lagerstelle 36 schwenkbar ist, als auch um eine in der Zeichenebene von Fig. 6 senkrecht zur optischen Achse der oberen Kamera 20 verlaufende Achse drehbar ist. Diese Schwenkbarkeit und Drehbarkeit dient dazu, die optische Achse der oberen Kamera 20 derart auszurichten, daß sich das Sehfeld der oberen Kamera 20 mit dem Sehfeld der unteren Kamera 18 im gewünschten und erforderlichen Ausmaß überdeckt. Bei richtiger Einstellung der oberen Kamera 20 wird die zweite Halterung 34 in ihrer Lage fixiert.

Bei dem hier beschriebenen Ausführungsbeispiel bilden die beiden Kameras 18 und 20 die Kameraeinrichtung der Vorrichtung. Obwohl als Kameras auf Film aufnehmende Kameras in Frage kommen, sind Videokameras vorzuziehen, und zwar insbesondere digitale Kameras, die eine Real-Time-Verarbeitung und -Bearbeitung ermöglichen. In Frage kommen hier Videokameras in CCD-Schaltungstechnik, CID-Schaltungstechnk und CMOS-Schaltungstechnik. Digitale Videokameras haben sich insbesondere im Hinblick auf die Genauigkeit der fotogrammetrischen Auswertung der Kamerasignale als überlegen erwiesen.

Obwohl bei dem hier beschriebenen Ausführungsbeispiel zwei Kameras die Kameraeinrichtung bilden, kann die Kameraeinrichtung auch aus lediglich einer Kamera oder drei Kameras bestehen. Im Falle von drei Kameras, die drei Aufnahmen machen, ist auch dann noch ein bestimmter Objektpunkt erkennbar und identifizerbar, wenn dieser lediglich in zwei der Aufnahmen erkennbar ist, in der dritten jedoch nicht. Wenn die Kameraeinrichtung lediglich eine Kamera umfaßt, so kann diese Kamera mit einer Vorrichtung versehen sein, die in der Bildebene der Kamera gleichzeitig zwei Bilder des Objektes erzeugt, und zwar zwei Bilder, die sich durch unterschiedliche Beobachtungsrichtungen unterscheiden. Eine solche Vorrichtung kann beispielsweise durch eine Spiegelanordnung gebildet sein. Schließlich ist es auch möglich, mit lediglich einer Kamera zu arbeiten und lediglich eine Aufnahme zu machen, wobei dann die rechnerische fotogrammetrische Auswertung aufwendiger wird, jedoch nicht unmöglich ist.

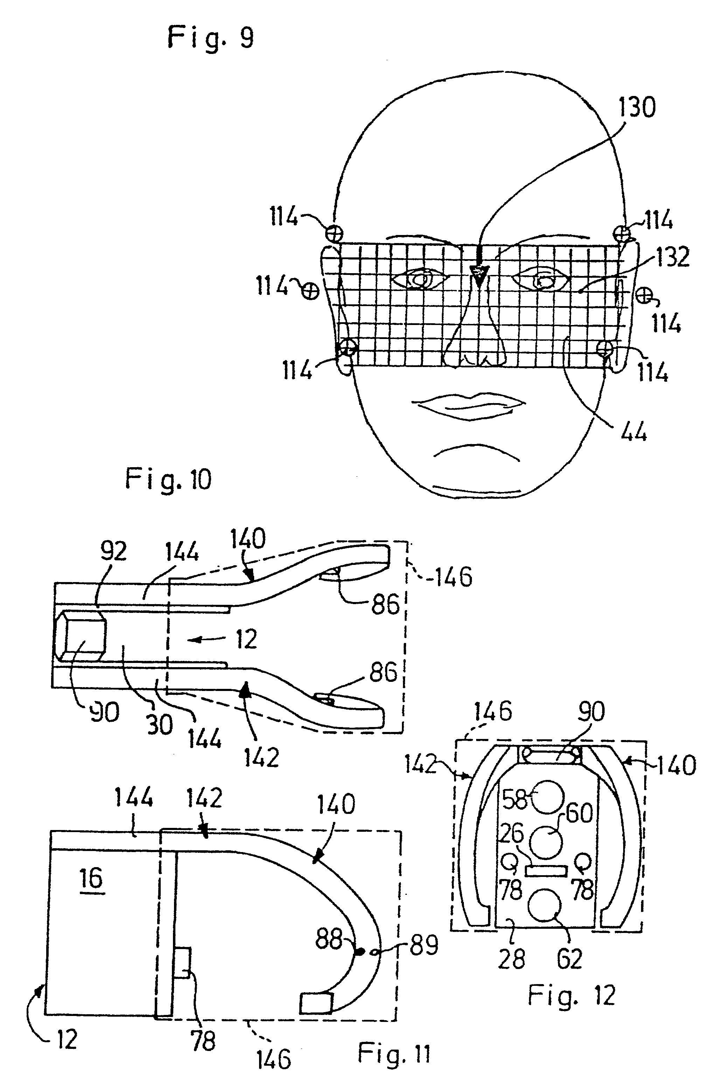

Zu den Halterungen des Geräteträgers 16 gehört ferner eine dritte Halterung 38, an der der Projektor 22 lösbar und austauschbar befestigt ist. Der Projektor 22 weist ein Objektiv 40 auf und trägt ein Diapositiv 42, das seinerseits die Vorlage für ein mittels des Projektors 22 zu projizierendes Musterbild 44 trägt, das schematisch in Fig. 9 dargestellt ist und beispielsweise das Bild eines Rechteckgitters ist. An der Halterung 38 für den Projektor 22 ist ein Stellantrieb 46 angebracht, mittels dessen auf nicht näher dargestellte Weise das Diapositv 42 im Projektor 22 um dessen optische Achse gedreht werden kann, so daß auf diese Weise auch das Musterbild 44 um eine zur Zeichenebene von Fig 9 senkrechte Achse gedreht werden kann.

Die Halterung 38 für den Projektor 22 ist um eine in der Zeichenebene von Fig 6 liegende und durch die Hauptebene des Objektivs 40 verlaufende Achse 48 schwenkbar. Diese Schwenkung wird mittels eines zweiten Stellantriebs 50 bewirkt, der an der Halterung 38 auf lediglich schematisch dargestellte Weise angreift. Durch Schwenken des Projektors 22 mittels des Stellantriebs 50 kann das vom Projektor 22 projizierte Musterbild 44 seitwärts verlagert werden, d.h. nach rechts bzw. links in Fig. 9.

In einer gewissen Entfernung vom optischen Kopf 12 befindet sich eine Meßebene 52, die in Fig 6 strichpunktiert angedeutet ist. In dieser Meßebene 52 soll sich das zu untersuchende Meßobjekt, nämlich der vordere Bereich des Kopfes 8 befinden, damit das Meßobjekt im Schärfetiefenbereich der beiden Kameras 18 und 20 sowie des Projektors 22 angeordnet ist. Auf diese Meßebene 52 sind dementsprechend sowohl das Objektiv 40 des Projektors 22 als auch die Objektive 54 und 56 der Kamera 18 bzw. 20 fokussiert. Ferner soll das Meßobjekt in der Meßebene 52 eine bestimmte Lage einnehmen, damit es sich etwa mittig in den Sehfeldern der beiden Kameras 18 und 20 befindet. Diese Lage des Meßobjektes in der Meßebene 52 wird als Meßstellung bezeichnet. Die optischen Achsen der beiden Objektive 56 und 54 sind nun derart ausgerichtet, daß das in der Meßstellung befindliche Meßobjekt mittels der beiden Kameras 18 und 20 aus voneinander abweichenden Richtungen scharf aufnehmbar ist. Der Fokussierzustand der beiden Kameras 18 und 20 sowie des Projektors 22 und die Ausrichtung der beiden Kameras 18 und 20 werden im Betrieb nicht geändert, so daß durch die eingestellten Fokussierzustände und Ausrichtungen die Lage der Meßebene 52 und die erforderliche Meßstellung des Kopfes 8 relativ zum optischen Kopf 12 festliegen. Eine der Besonderheiten der hier beschriebenen Vorrichtung ist nun, daß nicht der Kopf 8 aktiv in die Meßstellung bewegt wird, sondern der optische Kopf 12 - zusammen mit den mit ihm fest verbundenen Elementen - in senkrechter Richtung und horizontal verschoben wird, bis der Kopf 8 die Meßstellung einnimmt. Die Horizontalverschiebung findet dabei in der Zeichenebene von Fig. 6 statt, in der auch die optischen Achsen der beiden Kameras 18 und 20 liegen.

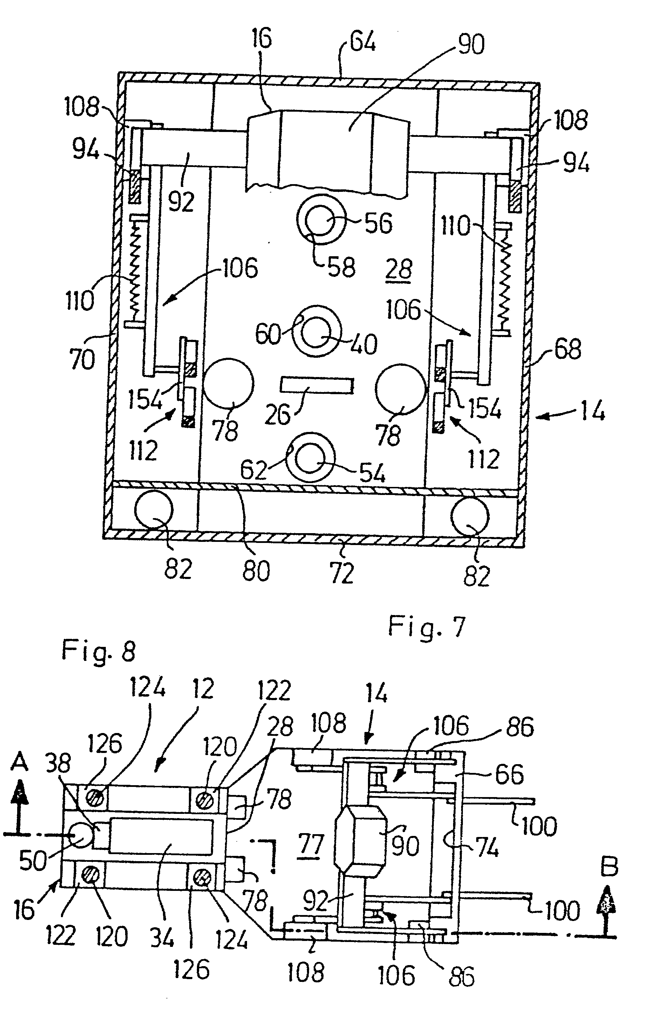

Wie die Fig. 6 und 7 erkennen lassen, weist die ebene Vorderwand 28 des Geräteträgers 16 drei Öffnungen 58, 60 und 62 auf, die in der Mitte der Vorderwand 28 senkrecht übereinander angeordnet sind. Hinter der Öffnung 58 ist das Objektiv 56 der oberen Kamera 20 angeordnet, hinter der Öffnung 60 ist das Objektiv 40 des Projektors 22 angeordnet, und hinter der Öffnung 62 ist das Objektiv 54 der unteren Kamera 18 angeordnet. Die Öffnungen 58, 60 und 62 sind derart dimensioniert, daß sie das Sehfeld der beiden Kameras und des Projektors jeweils nicht beeinträchtigen.

In der Mitte zwischen den beiden Öffnungen 60 und 62 weist die Frontwand 28 eine weitere Öffnung auf, in der die Linsenanordnung 26 befestigt ist. Die Linsenanordnung 26 und das Beobachtungsobjekt 24 sind derart im optischen Kopf 12 angeordnet und ausgebildet, daß dann, wenn der Kopf 8 seine Meßstellung einnimmt, die Person das Beobachtungsobjekt 24 mittels der Linsenanordnung 26 im Unendlichen oder in einer zumindest so großen Entfernung sieht, daß eine Nahpunktfixierung der Augen der Person vermieden ist.

Fest verbunden mit dem optischen Kopf 12 ist die Kammereinheit 14. Zu dieser gehört eie Haube, die durch eine obere Wand 14, die sich an die obere Wand 30 des Geräteträgers 16 anschließt, eine in Fig. 6 rechts angeordnete Frontwand, zwei Seitenwände 68 und 70 sowie durch eine Bodenwand 72 gebildet ist. In der Frontwand ist eine große Öffnung 74 ausgebildet, die eine solche Größe hat, daß bei in Meßstellung befindlichem Kopf 10 alle Bereiche desselben, die mittels der Kameras 18 und 20 aufgenommen werden sollen, für diese sichtbar sind. Die auf vorstehend beschriebene Weise ausgebildete Haube umschließt eine Kammer 77 und dunkelt den Raum zwischen der Vorderwand 28 des Geräteträgers 16 sowie der Frontwand 66 bzw. dem Meßobjekt ab, damit die Messungen nicht durch Störlicht beeinflußt werden. Die Frontwand 66 befindet sich dicht bei der Meßebene 52 und trägt einen die Öffnung 74 umschließenden Randwulst 76 aus weichem Kunststoffmaterial, der jeglicher Verletzung des Kopfes 8 vorbeugen soll, wenn es unbeabsichtigt zu einer Berührung zwischen dem Kopf und der Kammereinheit 14 kommen sollte.

Innerhalb der Kammer 77 sind an der Vorderwand 28 des Geräteträgers 16 beiderseits der Linsenanordnung 26 Lampen 78 angeordnet, die dazu dienen, den in Meßstellung befindlichen Kopf von vorne zu beleuchten, so daß sie gemeinsam eine Beleuchtungseinrichtung bilden. Außer dieser Beleuchtungseinrichtung weist die Vorrichtung eine Augenhöhlenbeleuchtungseinrichtung auf. Diese umfaßt beim dargestellten Ausführungsbeispiel zwei Lichtquellen 82, die unterhalb eines Zwischenbodens 80 der Kammereinheit 14 angeordnet sind. Jede der beiden Lichtquellen 82 sendet einen nach rechts in Fig. 6 gerichteten Lichtstrahl aus, der auf einen Spiegel 84 trifft, der den Lichtstrahl nach schräg oben umlenkt und durch eine Öffnung 86 im Zwischenboden 80 auf die Augenhöhlen des in Meßstellung befindlichen Kopfes richtet. Beim beschriebenen Ausführungsbeispiel sind für jede der beiden Augenhöhlen jeweils eine Lichtquelle 82, ein Spiegel 84 und eine Öffnung 86 vorgesehen. Durch Beleuchten der Augenhöhlen mittels der vorstehend beschriebenen Augenhöhlenbeleuchtungseinrichtung kann bei bestimmten Messungen bzw. Aufnahmen einer störenden Abschattung der Augenhöhlen vorgebeugt werden.

Die Vorrichtung ist ferner mit einer Entfernungsmeßeinrichtung versehen, die dazu dient, festzustellen, ob das Meßobjekt den erforderlichen Abstand vom optischen Kopf 12 hat, d.h. ob das Meßobjekt seine Meßstellung erreicht hat. Beim dargestellten Ausführungsbeispiel umfaßt diese Entfernungsmeßeinrichtung zwei Lichtschranken mit je einem optoelektronischen Empfänger und einem zugeordneten Sender, wobei die beiden Lichtschranken im wesentlichen horizontal quer zu den optischen Achsen der Kameras 18 und 20 verlaufen. Von den vorstehend genannten optoelektronischen Empfängern und Sendern ist lediglich ein Empfänger 88 in Fig. 6 erkennbar, der im Randwulst 76 angeordnet ist und somit von der Haube der Kammereinheit 14 getragen wird. Rechts vom Empfänger 88 ist ein weiterer Empfänger im Randwulst 76 angeordnet, wobei jedoch dieser weitere Empfänger in Fig. 6 vom Kopf 8 , d. h. von der Nasenwurzel des Kopfes, verdeckt ist. Diesen beiden Empfängern liegen senkrecht zur Zeichenebene von Fig. 6 zwei zugeordnete Sender gegenüber, die ebenfalls im Randwulst 76 angeordnet sind und somit von der Haube der Kammereinheit 14 getragen werden. Jedes Paar aus einem optoelektronischen Empfänger und einem Sender bildet eine Lichtschranke, wobei die Orte der Lichtschranken derart festgelegt sind, daß dann, wenn eine der beiden Lichtschranken vom Meßobjekt unterbrochen ist, die andere jedoch nicht, das Meßobjekt seine Meßstellung einnimmt. Nicht dargestellt in den Figuren sind die elektrischen Anschlüsse sowie die Auswertschaltung der beschriebenen Entfernungsmeßeinrichtung.

Innerhalb der Kammer 77 ist ein Kalibrierobjekt 90 angeordnet, das eine bestimmte, bekannte Form hat und dazu benutzt wird, die für die Kalibrierung der gesamten Vorrichtung erforderlichen Angaben zu gewinnen. Da das fotogrammetrische Meßverfahren jedoch nicht Gegenstand der Erfindung ist, wird das eigentliche Kalibrieren hier nicht weiter erläutert.

Das Kalibrierobjekt 90 ist an einem U-förmigen Bügel 92 befestigt, der zwei Schenkel 94 aufweist, die mit ihren freien Enden schwenkbar an den Seitenwänden 68 und 70 der Haube gelagert sind. Das Kalibrierobjekt 90 ist zwischen einer in Fig. 6 dargestellten Ruhestellung, in der es die freie Sicht der beiden Kameras 18 und 20 sowie des Projektors 22 auf das Meßobjekt nicht behindert, und einer Kalibrierstellung bewegbar, in der das Meßobjekt 90 in der Meßebene 52 angeordnet ist. Für genaue Positionierung des Kalibrierobjektes 90 sorgen Anschläge 96 an der Frontwand 66 der Haube. Sowohl in seiner Ruhestellung als auch in seiner Kalibrierstellung steht das Kalibrierobjekt 90 unter Federspannung, die von einer Feder 98 auf den Bügel 92 aufgebracht wird.

Das Kalibrierobjekt 90 weist mehrere ebene Flächen auf, die voneinander unterschiedliche Ausrichtungen im Raum haben und mit Kalibriermarken versehen sind. Eine der ebenen Fläche des Kalibrierobjektes 90 ist in dessen Kalibrierstellung der Kameraeinrichtung zugewandt und während des Kalibirierens vorzugsweise genau senkrecht (d.h. zum Erdmittelpunkt) ausgerichtet, so daß durch das Kalibrierobjekt in seiner Kalibrierstellung eine von der Vorrichtung unabhängige, absolute Ausrichtung des Kalibrierobjektes im Raum vorgegeben wird.

Obwohl beim beschriebenen Ausführungsbeispiel das Kalibrierobjekt vorzugsweise ständig mittelbar oder unmittelbar am optischen Kopf 12 angebracht ist, kann es auch von diesem lösbar sein, beispielsweise lediglich zum Zweck der Kalibrierung am optischen Kopf 12 bzw. der Kammereinheit 14 eingehängt werden und ansonsten vom Geräteträger 16 getrennt sein.

In Fig. 6 ist ein Ohrtaster 100 erkennbar, und zwar der Ohrtaster für das linke Ohr der Person. Dieser Ohrtaster weist einen im wesentlichen horizontal verlaufenden Abschnitt 148 sowie an dessen in Fig. 6 rechtem Ende einen sichelförmig gekrümmten Abschnitt 102 auf, der aus einem weichen, bandförmigen Material besteht und ungefähr dem Verlauf des Bereichs zwischen dem Ohr und dem Schädel eines Menschen nachgeformt ist. Dieser weiche Abschnitt 102 ist an seinem oberen Ende mit dem waagerechten Abschnitt 148 des Ohrtasters 100 fest verbunden und wird an seinem unteren Ende von einem steiferen, ebenfalls sichelförmigen Abschnitt 104 gehalten, der nicht in Kontakt mit dem Ohr oder dem Schädel stehen soll. Der vorstehend beschriebene Ohrtaster 100 ist gelenkig mit einer Parallelführungseinrichtung 106 verbunden, die beim dargestellten Ausführungsbeispiel aus zwei miteinander gekoppelten Parallelogrammführungen 112 und 150 besteht, wobei ein Ende der Parallelführungseinrichtung 106 mittels eines Blocks 108 an der Seitenwand 70 befestigt ist. Die obere Parallelogrammführung 150 umfaßt zwei parallel zueinander verlaufende Streben 152, die mit ihren oberen Enden am Block 108 schwenkbar angelenkt sind. Die beiden unteren Enden der Streben 152 sind gelenkig mit einer Platte 154 verbunden, wobei die vier Anlenkpunkte der beiden Streben 152 an den Ecken enes Parallelogramms angeordnet sind. Auch die untere Parallelogrammführung 112 umfaßt zwei Streben 156, die parallel zueinander verlaufen und die einerseits mit ihren einen Enden schwenkbar an der Platte 154 angelenkt sind und andererseits mit ihren anderen Enden gelenkig mit dem Ohrtaster 100 verbunden sind, wobei auch die vier Anlenkpunkte der Streben 156 an den Ecken eines Parallelogramms liegen. In Anbetracht dessen, daß der Ohrtaster im wesentlichen horizontal aus der Kammereinheit 14 heraus- und in diese hineinbewegbar sein soll, ist die obere Parallelogrammführung 150 vorzugsweise derart angeordnet, daß die Streben 152 im wesentlichen senkrecht verlaufen, während sie ein Rechteck bilden, wogegen die Streben 156 der unteren Parallelogrammführung 112 vorzugsweise dann ein Rechteck bilden, wenn sie im wesentlichen horzontal verlaufen. Ein Ohrtaster 100 und eine Parallelführungs einrichtung 106, wie sie vorstehend für das linke Ohr beschrieben sind, sind auch für das rechte Ohr vorgesehen, wie Fig. 7 erkennen läßt, wobei die beiden Ohrtaster und die beiden Parallelführungseinrichtungen symmetrisch zueinander ausgebildet sind. Jede Parallelführungseinrichtung 106 ist mit einer Feder 110 versehen, die den jeweiligen Ohrtaster 100 nach links in Fig. 6 zu ziehen versucht. Die Parallelführungseinrichtung 106 ermöglicht es, daß der jeweilige Ohrtaster 100 parallel zu sich selbst nach oben und unten sowie vorne und hinten, d.h. nach links und rechts in Fig. 6, verschoben wird und auf diese weise in Kontakt mit der Rille zwischen der Ohrmuschel und dem Schädel steht. jeder Ohrtaster ist mit drei lediglich in Fig. 9 dargestellten Marken versehen, die für die beiden Kameras 18 und 20 sichtbar sind und bei an die Ohren des die Meßstellung einnehmenden Kopfes angelegten Ohrtastern 100 ungefähr in der Meßebene 52 angeordnet sind. Die Marken 114 werden zusammen mit dem auf das Gesicht projizierten Musterbild von den beiden Kameras 18 und 20, die zusammen die Kameraeinrichtung der Vorrichtung bilden, aufgenommen und ermöglichen es, Lage und Ausrichtung des Ohrtasters 100 im Raum und somit des Übergangsbereichs zwischen Ohr und Schädel anhand der Aufnahmen zu bestimmen. Die Gelenke der unteren Parallelogrammführung 112 der Parallelführungseinrichtung 106 sind vorzugsweise als Kugelgelenke ausgebildet, damit der Ohrtaster in gewissen Grenzen auch eine Drehung um die Längsachse seines geraden, horizontal verlaufenden Abschnitts ausführen kann.

Bei dem vorstehend beschriebenen Ausführungsbeispiel sind die Parallelführungseinrichtungen 106 der beiden Ohrtaster 100 an der Kammereinheit 14 und somit mittelbar am Geräteträger 16 des optischen Kopfes 12 an gebracht. Die beiden Parallelführungseinrichtungen können jedoch auch an der Sitzgelegenheit 6 bzw. vorzugsweise an deren Kopfstütze 10 angebracht sein, wobei dann die Ohrtaster 100 von hinten an die Ohren angelegt und angedrückt werden, statt daß die Ohrtaster von ihren Parallelführungseinrichtungen 106 nach vorne gezogen werden, wie dies beim dargestellten Ausführungsbeispiel der Fall ist.

Wie sich aus der vorstehenden Beschreibung ergibt, sind der optische Kopf 12 und die Kammereinheit 14 gemeinsam relativ zum vorrichtungsfesten Unterbau 2 in senkrechter Richtung und in der durch die optischen Achsen der Kameras 18 und 20 definierten Horizontalrichtung bewegbar. Zu diesem Zweck befindet sich im Unterbau 2 ein nicht dargestellter Schlitten, der in der genannten Horizontalrichtung verfahrbar ist und dem eine ebenfalls nicht dargestellte elektromotorische Antriebseinrichtung zugeordnet ist. In der Oberseite des Unterbaus 2 ist ein Schlitz 116 ausgebildet, in dessen Richtung und unterhalb desselben der nicht dargestellte Schlitten verfahren wird. Der freie Bereich des Schlitzes 116 ist durch eine Jalousie 118 abgedeckt. Der nicht dargestellte Schlitten trägt eine Führungseinrichtung zum Führen des Geräteträgers 16 am Schlitten bei seiner senkrechten Bewegung. Von dieser Führungseinrichtung sind lediglich in Fig. 8 zwei senkrechte Führungsstangen 120 erkennbar, die am nicht dargestellten Schlitten befestigt sind und durch Führungsbuchsen 122 verlaufen, die am Geräteträger 16 befestigt sind, und zwar im Bereich von zwei diagonal gegenüberliegenden Kanten des quaderförmigen Geräteträgers. Parallel zu den beiden übrigen Kanten verlaufen zwei Antriebsspindeln 124, die drehbar im nicht dargestellten Schlitten gelagert und axial an diesem abgestützt sind. Die beiden Antriebsspindeln 124 stehen in Eingriff mit jeweils einer Mutter 126, die am Geräteträger 16 befestigt ist. Den beiden Antriebsspindeln ist eine nicht dargestellte elektromotorische Antriebseinrichtung zugeordnet, mit deren Hilfe die Antriebsspindeln gedreht werden können, um auf diese Weise den optischen Kopf 12 zusammen mit der Kammereinheit 14 aufwärts oder abwärts zu verfahren. Die Führungsstangen 120 und die Antriebsspindeln 124 sind unterhalb des optischen Kopfes 12 von einer teleskopischen Abdeckung 128 umschlossen.

Vorstehend nicht erläutert und nicht dargestellt sind die elektrischen Anschlüsse und Steuervorrichtungen der Kameras 18 und 20, des Projektors 22, der Lichtschranken, der verschiedenen elektromotorischen Antriebsvorrichtungen sowie der Stellantriebe 46 und 50, da diese elektrischen Anschlüsse und Steuervorrichtungen zum Verständnis der Erfindung nicht notwendig sind.

Die vorstehend beschriebene Vorrichtung arbeitet in folgender Weise. Eine Person, deren Gesicht vermessen werden soll, nimmt auf der Sitzgelegenheit 6 Platz und wird aufgefordert, eine bequeme, aufrechte Sitzposition einzunehmen und dabei den Kopf im wesentlichen gerade zu halten und geradeaus nach vorne, d.h. in die Öffnung 74 zu blicken. Nachdem die Person bestätigt hat, daß sie diese Position eingenommen hat, wird die Kopfstütze 10 von hinten an den Kopf 8 angelegt, um es der Person zu erleichtern, den Kopf 8 in der eingenommenen Stellung zu halten. Danach werden dann mittels einer nicht dargestellten Steuervorrichtung die elektromotorischen Stellantriebe für die Horizontalbewegung und Vertikalbewegung des optischen Kopfes 12 derart betätigt, daß der optische Kopf 12 horizontal und/oder vertikal verfahren wird, bis der Kopf 8 seine Meßstellung einnimmt. Zu einem Zeitpunkt, zu dem der Abstand des Kopfes 8 vom optischen Kopf 12 noch größer als in der Meßstellung ist, ist bereits auf einer nicht dargestellten Bildschirmanordnung, auf der die von den beiden Kameras 18 und 20 aufgenommenen Bilder wiedergegeben werden, ein pfeilförmiges Markenbild 130 zu erkennen, dessen Vorlage sich auf dem Diapositiv 42 vorzugsweise derart befindet, daß es auf der optischen Achse des Objektivs 40 des Projektors 22 liegt. Der optische Kopf 12 wird dann in eine solche Höhe gefahren, daß das Markenbild 130 in Höhe der Nasenwurzel auf den Nasenrücken projiziert ist, wie dies in Fig. 9 gezeigt ist. Zu diesem Zeitpunkt oder bereits vorher werden die beiden Ohrtaster 100 aus der Kammer 77, in der sie bis dahin abgelegt waren, soweit herausgezogen, daß die weichen Abschnitte 102 von hinten in die Rille zwischen den Ohrmuscheln und dem Schädel mit geringem Druck gelegt werden können. Danach wird dann der optische Kopf 12 weiter auf das Meßobjekt bzw. Gesicht zugefahren, bis dieses seine Meßstellung einnimmt. Wenn die Meßstellung erreicht ist, spricht die Entfernungsmeßeinrichtung in Form der beiden Lichtschranken an, woraufhin entweder manuell oder automatisch die Vorwärtsbewegung des optischen Kopfes 12 zusammen mit der Kammereinheit 14 beendet wird. Falls dies notwendig sein sollte, erfolgt danach noch eine Korrektur der Vertikalposition des optischen Kopfes 12, bis das Markenbild 130 die vorstehend erläuterte, gewünschte Höhe hat. Mittels des Stellantriebes 50 wird danach der Projektor 22 um die Achse 48 geschwenkt, bis das Markenbild 130 mittig auf dem Nasenrücken liegt, sofern dies nicht schon vorher der Fall war. Schließlich wird das Diapositiv 42 gedreht, bis eine Bezugslinie 132 des Musterbildes 44 eine gewünschte Sollage hat, beispielsweise den äußeren Irisrand beider Augen berührt, wie dies in Fig. 9 gezeigt ist. Wenn dann schließlich der in Fig. 9 gezeigte Zustand erreicht ist, können mittels der aus den Kameras 18 und 20 bestehenden Kameraeinrichtung gleichzeitig die zwei Aufnahmen gemacht werden, die dann einer fotogrammetrischen Auswertung unterworfen werden. Ohne aufprojiziertes Musterbild 44 und aufprojiziertes Markenbild sowie bei abgenommenen Ohrtastern 100 und eingeschalteten Lampen 78 kann außerdem beispielsweise mittels der Kamera 18 eine weitere Aufnahme des Gesichtes gemacht werden, die ein Vergleichsbild liefern soll.

Nachdem die gewünschten Aufnahmen gemacht worden sind, werden dann der optische Kopf 12 und die Kammereinheit 14 vom Kopf 8 weggefahren und ggf. in eine Ruhestellung bezüglich des Unterbaus 2 gebracht.

Bei dem vorstehend beschriebenen Ausführungsbeispiel erfolgt die Querpositionierung durch Schwenken des Projektors um die Achse 48. Es versteht sich, daß diese Querpositionierung allein oder zusätzlich auch dadurch bewirkt werden könnte, daß der optische Kopf 12 zusammen mit der Kammereinheit 14 in einer zweiten Horzontalrichtung relativ zum vorrichtungsfesten Unterbau 2 verfahren wird, die senkrecht zur ersten Horizontalrichtung verläuft. Bei vorstehend beschriebenem Ausführungsbeispiel erfolgt die vertikale Positonierung ausschließlich durch Verfahren des optischen Kopfes 12 in senkrechter Richtung. Alternativ könnte zusätzlich vorgesehen sein, daß durch entsprechende Bewegung des Projektors 22 bzw. des Diapositivs 42 das Musterbild 44 und das Markenbild 130 in Fig. 9 in senkrechter Richtung verlagert werden. Schließlich ist es auch möglich, den gesamten Projektor 22 um die optische Achse seines Objektivs 40 zu drehen, statt das Diapositiv 42 im Projektor 22 zu drehen.

Die in den Fig. 3, 4 und 5 dargestellte zweite Ausführungsform der Vorrichtung unterscheidet sich hinsichtlich der Ausbildung des optischen Kopfes 12 und der Kammereinheit 14 nicht von der vorstehend beschriebenen Ausführungsform, weswegen die diesbezüglichen Erläuterungen nicht erneut gegeben werden. Bei der Ausführungsform gemäß den Fig. 3 bis 5 sind am plattenförmigen Unterbau 2 zwei senkrechte Säulen 132 und 134 befestigt, die an ihren oberen Enden durch ein Joch 136 miteinander verbunden sind. An den Säulen 132 und 134 ist senkrecht verfahrbar ein Schlitten 138 geführt, der bei seiner senkrechten Bewegung mittels einer nicht dargestellten elektromotorischen Antriebsvorrichtung angetrieben wird. Der Schlitten 138 trägt den optischen Kopf 12 derart, daß der Geräteträger 16 des optischen Kopfes 12 am Schlitten 138 in der ersten Horizontalrichtung verfahrbar ist, wobei diese Bewegung mittels einer nicht dargestellten elektromotorischen Antriebseinrichtung bewirkt wird.

Die in den Fig 10, 11 und 12 dargestellte dritte Ausführungsform unterscheidet sich von der ersten Ausführungsform im wesentlichen durch das Fehlen der Kammereinheit 14 der ersten Ausführungsform. Der optische Kopf 12 der dritten Ausführungsform hat die gleiche Ausbildung wie die optischen Köpfe der ersten und zweiten Ausführungsformen, weswegen er nicht erneut erläutert wird. Der optische Kopf 12 der dritten Ausführungsform kann entweder wie bei der ersten oder wie bei der zweiten Ausführungsform mit dem Unterbau verbunden und von diesem getragen sein. Der Unterbau und die den optischen Kopf 12 mit dem Unterbau verbindenden Einrichtungen sind für die dritte Ausführungsform nicht dargestellt. Die Parallelführungseinrichtungen der Ohrtaster sind bei der dritten Ausführungsform an der in den Fig 10, 11 und 12 nicht dargestellten Sitzgelegenheit angebracht.

Bei der Ausführungsform gemäß den Fig 10 bis 12 sind auf der oberen Wand 30 des Geräteträgers 16 ein Arm 140 sowie ein zweiter Arm 142 befestigt. Wie die Fig. 10 und 12 zeigen, sind die beiden Arme 140 und 142 symmetrisch zur Längsmittelebene des optischen Kopfes 12 ausgebildet. Jeder der beiden Arme hat einen unmittelbar am Geräteträger befestigten, geraden Abschnitt 144, wobei an den beiden geraden Abschnitten 144 der Bügel 92 mit dem Kalibrierobjekt 90 schwenkbar gelagert ist. Nach unten und etwas nach außen gekrümmt verläuft jeder Arm 140 vom Abschnitt 144 aus bis zu den Orten der Sender bzw. Empfänger der die Entfernungsmeßeinrichtung bildenden Lichtschranken, wobei in Fig. 11 außer dem Empfänger 88 auch der neben diesem angeordnete Empfänger 89 erkennbar ist, weil er in Fig. 11 nicht durch den Kopf 8 abgedeckt ist. Im Arm 142 befinden sich an den den Empfängern 88 und 89 entsprechenden Orten die zugeordneten Sender.

Die beiden Arme 140 und 142 verlaufen unterhalb der Sender bzw. Empfänger der Lichtschranken bogenförmig in Richtung zum optischen Kopf 12 und weisen schließlich ein freies Ende auf. Nahe den freien Enden sind in den Armen 140 und 142 die Elemete der Augenhöhlenbeleuchtungseinrichtung angeordnet, vor der in Fig. 10 lediglich die Öffnungen 86 erkennbar sind.

Die Ausführungsform gemäß den Fig. 10 bis 12 hat den Vorteil, daß die Person, deren Kopf vermessen werden soll, nicht durch die verhältnismäßig große Kammereinheit 14 mit der dunklen Öffnung 74 der ersten Ausführungsform irritiert wird, während der optische Kopf 12 und die Kammereinheit 14 vorbewegt werden. Die Ausführungsform gemäß den Fig. 10 bis 12 eignet sich besonders dann, wenn durch die Beleuchtungsverhältnisse in dem Raum, in dem die Vorrichtung aufgestellt ist, dafür gesorgt ist, daß die Fotogrammetrie durch Störlicht nicht beeinträchtigt wird. Darüber hinaus kann für diese Ausführungsform eine gestrichelt dargestellte Abdeckung 146 vorgesehen sein, die ungefähr die Form der Haube der ersten Ausführungsform hat und ebenfalls ihr Inneres abdunkelt. Die Abdeckung 146 ist auf einfache Weise aufsetzbar und abnehmbar und kommt dann zur Anwendung, wenn die vorstehend genannte Forderung hinsichtlich der Störlichtbeeinflussung nicht erfüllt ist.

Die Arbeitsweise der zweiten und dritten Ausführungsform der Vorrichtung stimmt mit der Arbeitsweise der ersten Ausführungsform überein, weswegen sie nicht erneut erläutert wird.

Es versteht sich, daß zahlreiche Abwandlungen der vorstehend beschriebenen Ausführungsbeispiele möglich sind, ohne den Rahmen der Erfindung zu verlassen.

**Patentansprüche**

1. Vorrichtung zum fotogrammetrischen Erfassen des menschlichen Kopfes, mit einem Projektor zum Projizieren eines Musterbildes, einer Kameraeinrichtung, die zumindest eine Aufnahme des Kopfes mit dem darauf projizierten Musterbild aufnehmen kann, und mit einer Sitzgelegenheit für die Person, deren Kopf fotogrammetrisch erfaßt werden soll, gekennzeichnet durch einen vorrichtungsfesten Unterbau (2) und einen Geräteträger (16), der sowohl die Kameraeinrichtung (18, 20) als auch den Projektor (22) trägt, wobei der Geräteträger (16) und der Kopf relativ zueinander in senkrechter Richtung (X) und in einer Horizontalrichtung (Y), die im wesentlichen mit der optischen Achse der Kameraeinrichtung zusammenfällt, bewegbar sind, zwei Ohrtaster (100), die jeweils an den Bereich zwischen einem der Ohren und dem Schädel des Kopfes anlegbar sind und mit Marken (114) versehen sind, die bei angelegtem Ohrtaster zugleich mit der Vorderseite des Kopfes sichtbar sind, und eine Parallelführungseinrichtung (106) für jeden der Ohrtaster, die eine Parallelverschiebung des jeweiligen Ohrtasters in der senkrechten Richtung und in der genannten Horizontalrichtung ermöglicht, wobei die Parallelführungseinrichtungen an der Sitzgelegenheit (6) oder am Geräteträger (16) angebracht sind.

2. Vorrichtung nach Anspruch 1, dadurch gekennzeichnet, daß der Geräteträger (16) in der senkrechten Richtung (X) und in der einen Horizontalrichtung (Y) relativ zum vorrichtungsfesten Unterbau (2) bewegbar ist.

3. Vorrichtung nach Anspruch 2, dadurch gekennzeichnet, daß der Geräteträger (16) in einer zweiten Horizontalrichtung bewegbar ist, die im wesentlichen senkrecht zur ersten Horizontalrichtung (Y) verläuft.

4. Vorrichtung nach einem der Ansprüche 1 bis 3, gekennzeichnet durch einen Schlitten, der in der ersten Horizontalrichtung verfahrbar am Unterbau (2) geführt ist und den Geräteträger (16) trägt.

5. Vorrichtung nach Anspruch 4, gekennzeichnet durch zumindest eine Antriebsspindel (124), die im wesentlichen senkrecht verlaufend axial am Schlitten abgestützt ist und in Eingriff mit einer am Geräteträger (16) festen Mutter (126) steht, und eine Führungseinrichtung (120, 122) zum Führen des Geräteträgers am Schlitten bei seiner senkrechten Bewegung.

6. Vorrichtung nach Anspruch 1, gekennzeichnet durch eine am Unterbau (2) feste senkrechte Säule (132) und einen an der Säule senkrecht bewegbar geführten Schlitten (138), der den Geräteträger (16) derart trägt, daß er am Schlitten in der ersten Horizontalrichtung verfahrbar ist.

7. Vorrichtung nach einem der Ansprüche 1 bis 6, gekennzeichnet durch elektromotorische Antriebseinrichtungen für die Bewegungen des Geräteträgers (16) in den ihm möglichen Bewegungsrichtungen.

8. Vorrichtung nach einem der Ansprüche 1 bis 7, gekennzeichnet durch eine Halterung (38) für den Projektor (22) im Geräteträger (16), die eine Schwenkung des Projektors in der Weise ermöglicht, daß dabei das projizierte Musterbild (44) horizontal verlagert wird, und einen Stellantrieb (50) zum Schwenken der Halterung.

9. Vorrichtung nach einem der Ansprüche 1 bis 8, mit einem in den Projektor eingesetzten Diapositiv, dadurch gekennzeichnet, daß das Diapositiv (42) im Projektor (22) um dessen optische Achse drehbar ist und daß ein Stellantrieb (46) zum Drehen des Diapositivs vorgesehen ist.

10. Vorrichtung nach einem der Ansprüche 1 bis 9, dadurch gekennzeichnet, daß mittels der Kameraeinrichtung (18, 20) gleichzeitig aus verschiedenen Richtungen zwei Aufnahmen des Kopfes mit dem darauf projizierten Musterbild aufnehmbar sind.

11. Vorrichtung nach Anspruch 10, dadurch gekennzeichnet, daß die Kameraeinrichtung eine Kamera aufweist, die mit einer Vorrichtung versehen ist, die die den zwei Aufnahmen zugeordneten Bilder in zwei verschiedenen Bereichen der Bildebene der Kamera erzeugt.

12. Vorrichtung nach Anspruch 10, dadurch gekennzeichnet, daß die Kameraeinrichtung zwei Kameras (18, 20) umfaßt.

13. Vorrichtung nach Anspruch 12, dadurch gekennzeichnet, daß die eine Kamera (20) oberhalb und die andere Kamera (18) unterhalb des Projektors (22) angeordnet ist.

14. Vorrichtung nach einem der Ansprüche 10 bis 12, dadurch gekennzeichnet, daß jede Kamera (18, 20) als Videokamera ausgebildet ist.

15. Vorrichtung nach Anspruch 14, dadurch gekennzeichnet, daß jede Kamera (18, 20) eine digitale Videokamera ist.

16. Vorrichtung nach einem der Ansprüche 1 bis 15, gekennzeichnet durch ein vom Geräteträger (16) getragenes Beobachtungsobjekt (24) und eine dem Beobachtungsobjekt zugeordnete Linsenanordnung (26), die das Beobachtungsobjekt in großer Entfernung erscheinen läßt.

17. Vorrichtung nach einem der Ansprüche 1 bis 16, gekennzeichnet durch eine Entfernungsmeßeinrichtung (88, 89) zur Messung der Entfernung des Kopfes von der Kameraeinrichtung (18, 20) und dem Projektor (22).

18. Vorrichtung nach Anspruch 17, dadurch gekennzeichnet, daß die Entfernungsmeßeinrichtung zwei Lichtschranken mit je einem optoelektronischen Empfänger (88, 89) und einem zugeordneten Sender umfaßt, wobei die beiden Lichtschranken im wesentlichen horizontal quer zur optischen Achse der Kameraeinrichtung (18, 20) verlaufen.

19. Vorrichtung nach einem der Ansprüche 1 bis 18, gekennzeichnet durch eine am Geräteträger (16) angeordnete Beleuchtungseinrichtung (78) zur Beleuchtung des Kopfes im wesentlichen von vorn.

20. Vorrichtung nach einem der Ansprüche 1 bis 19, gekennzeichnet durch eine Augenhöhlenbeleuchtungseinrichtung (82, 84, 86), die zwei nach schräg oben gerichtete Beleuchtungslichtstrahlen aussendet.

21. Vorrichtung nach einem der Ansprüche 1 bis 20, gekennzeichnet durch ein Kalibrierobjekt (90), das in eine Kalibrierstellung und eine Ruhestellung bewegbar ist, wobei das Kalibrierobjekt in seiner Kalibrierstellung im wesentlichen die Position des Kopfes während der Messung einnimmt und in seiner Ruhestellung außerhalb des Sehfeldes der Kameraeinrichtung (18, 20) angeordnet ist.

22. Vorrichtung nach Anspruch 21, dadurch gekennzeichnet, dar das Kalibrierobjekt (90) am Geräteträger (16) gelagert ist.

23. Vorrichtung nach einem der Ansprüche 1 bis 22, gekennzeichnet durch eine am Geräteträger (16) befestigte Haube mit zwei Seitenwänden (68, 70), einer Bodenwand (72), einer oberen Wand (64) und einer Frontwand (66), wobei die Frontwand mit einer Öffnung (74) für den Kopf versehen ist und vom Geräteträger ungefähr denjenigen Abstand hat, den während der Messung der Kopf vom Geräteträger hat, und wobei die Haube den Raum zwischen der Kameraeinrichtung (18, 20) und dem Projektor (22) einerseits sowie der Frontwand (66) andererseits nach außen abdunkelt.

24. Vorrichtung nach Anspruch 23 in Verbindung mit einem der Ansprüche 17 und 18 sowie in Verbindung mit den Ansprüchen 20 und 22, dadurch gekennzeichnet, daß die Entfernungsmeßeinrich-tung (88, 89), die Augenhöhlenbeleuchtungseinrich-tung (82, 84, 86), das Kalibrierobjekt (90) und die zwei Parallelführungseinrichtungen (106) der Ohrtaster (100) an bzw. in der Haube (64, 66, 68, 70, 72) angeordnet sind.

25. Vorrichtung nach Anspruch 18, gekennzeichnet durch zumindest einen am Geräteträger (16) befestigten Arm (140, 142), der sich bis zu den Orten der zwei Sender und zwei Empfänger (88, 89) erstreckt und die Sender und Empfänger trägt.

26. Vorrichtung nach den Ansprüchen 25 und 20, dadurch gekennzeichnet, daß der zumindest eine Arm (140, 142) ferner die Augenhöhlenbeleuchtungseinrichtung (86) trägt.

27. Vorrichtung nach Anspruch 25 oder 26, gekennzeichnet durch eine lösbar auf den zumindest einen Arm (140, 142) und den Geräteträger (16) aufsetzbare Abdeckung (146), die den Raum zwischen der Kameraeinrichtung (18, 20) und dem Projektor (22) einerseits sowie der Entfernungsmeßeinrichtung (88, 89) andererseits abdunkelt.

## Claims

1. Device for photogrammetrically recording the human head, having a projector for the projection of a specimen image, a camera device, which can record at least one recording of the head with the specimen image projected thereon, and having a seating accommodation for the person whose head is to be photogrammetrically recorded, characterized by a base (2) fixed to the device and a systems carrier (16), which carries both the camera device (18, 20) and also the projector (22), the systems carrier (16) and the head being movable relative to one another in a vertical direction (X) and in a horizontal direction (Y), which substantially coincides with the optical axis of the camera device, two ear probes (100), which can be applied in each instance to the region between one of the ears and the skull of the head and are provided with marks (114), which, when the ear probe is applied, are visible together with the front side of the head, and a parallel guide device (106) for each one of the ear probes, which permits a parallel displacement of the respective ear probe in the vertical direction and in the said horizontal direction, the parallel guide devices being fitted to the seating accommodation (6) or to the systems carrier (16).

2. Device according to Claim 1, characterized in that the systems carrier (16) is movable in the vertical direction (X) and in the one horizontal direction (Y) relative to the base (2) fixed to the device.

3. Device according to Claim 2, characterized in that the systems carrier (16) is movable in a second horizontal direction, which extends substantially perpendicularly to the first horizontal direction (Y).

4. Device according to one of Claim(s) 1 to 3, characterized by a carriage, which is guided on the base (2) so as to be movable in the first horizontal direction and carries the systems carrier (16).

5. Device according to Claim 4, characterized by at least one drive spindle (124), which is supported axially on the carriage to extend substantially vertically and is in engagement with a nut (126) fixed to the systems carrier (16), and a guide device (120, 122) to guide the systems carrier on the carriage in the course of its vertical movement.

6. Device according to Claim 1, characterized by a vertical column (132) fixed to the base (2) and a carriage (138), which is guided to be vertically movable on the column and which carries the systems carrier (16) in such a manner that it is movable on the carriage in the first horizontal direction.

7. Device according to one of Claim(s) 1 to 6, characterized by electric motor drive devices for the movements of the systems carrier (16) in the directions of movement which are possible for it.

8. Device according to one of Claim(s) 1 to 7, characterized by a mounting (38) for the projector (22) in the systems carrier (16), which mounting permits a pivoting of the projector in such a manner that in this case the projected specimen image (44) is horizontally displaced, and a setting drive (50) to pivot the mounting.

9. Device according to one of Claim(s) 1 to 8, having a slide inserted into the projector, characterized in that the slide (42) is rotatable in the projector (22) about its optical axis, and in that a setting drive (46) is provided for the rotation of the slide.

10. Device according to one of Claim(s) 1 to 9, characterized in that by means of the camera device (18, 20) two recordings of the head with the specimen image projected thereon can be recorded simultaneously from different directions.

11. Device according to Claim 10, characterized in that the camera device exhibits a camera which is provided with a device which generates the images associated with the two recordings in two different regions of the image plane of the camera.

12. Device according to Claim 10, characterized in that the camera device comprises two cameras (18, 20).

13. Device according to Claim 12, characterized in that one of the cameras (20) is disposed above and the other camera (18) is disposed below the projector (22).

14. Device according to one of Claim(s) 10 to 12, characterized in that each camera (18, 20) is designed as a video camera.

15. Device according to Claim 14, characterized in that each camera (18, 20) is a digital video camera.

16. Device according to one of Claim(s) 1 to 15, characterized by an observation object (24) carried by the systems carrier (16) and a lens arrangement (26), which is associated with the observation object and which permits the observation object to appear at a large distance.

17. Device according to one of Claim(s) 1 to 16, characterized by a range finder (88, 89) for the measurement of the distance of the head from the camera device (18, 20) and the projector (22).

18. Device according to Claim 17, characterized in that the range finder comprises two light barriers with a respective optoelectronic receiver (88, 89) and an associated emitter, the two light barriers extending substantially horizontally transversely to the optical axis of the camera device (18, 20).

19. Device according to one of Claim(s) 1 to 18, characterized by an illumination device (78), disposed on the systems carrier (16), for the illumination of the head substantially from the front.

20. Device according to one of Claim(s) 1 to 19, characterized by an eye cavity illumination device (82, 84, 86), which emits two obliquely upwardly directed illumination light beams.

21. Device according to one of Claim(s) 1 to 20, characterized by a calibration object (90), which is movable into a calibration position and a rest position, the calibration object in its calibration position adopting substantially the position of the head during the measurement and being disposed, in its rest position, outside the field of view of the camera device (18, 20).

22. Device according to Claim 21, characterized in that the calibration object (90) is mounted on the systems carrier (16).

23. Device according to one of Claim(s) 1 to 22, characterized by a hood, secured to the systems carrier (16), with two side walls (68, 70), a base wall (72), an upper wall (64) and a front wall (66), the front wall being provided with an opening (74) for the head and having from the systems carrier approximately that spacing which the head has from the systems carrier during the measurement, and the hood darkening off outwardly the space between the camera device (18, 20) and the projector (22) on the one hand and the front wall (66) on the other hand.

24. Device according to Claim 23 in conjunction with one of Claim(s) 17 and 18, as well as in conjunction with Claim(s) 20 and 22, characterized in that the range finder (88, 89), the eye cavity illumination device (82, 84, 86), the calibration object (90) and the two parallel guide devices (106) of the ear probes (100) are disposed on or in the hood (64, 66, 68, 70, 72).

25. Device according to Claim 18, characterized by at least one arm (140, 142), which is secured to the systems carrier (16) and which extends to the locations of the two emitters and two receivers (88, 89) and carries the emitters and receivers.

26. Device according to Claim(s) 25 and 20, characterized in that at least one arm (140, 142) further carries the eye cavity illumination device (86).

27. Device according to Claim 25 or 26, characterized by a cover (146), which can be releasably fitted on the at least one arm (140, 142) and the systems carrier (16) and which darkens off the space between the camera device (18, 20) and the projector (22) on the one hand and the range finder (88, 89) on the other hand.

**Revendications**

1. Dispositif pour la saisie photogrammétrique de la tête humain, comportant un projecteur pour projeter une image modèle, un dispositif photographique qui peut prendre au moins un cliché de la tête avec l'image modèle projetée sur lui, et un siège pour la personne dont on veut saisir photographiquement la tête, caractérisé par un socle (2) solidaire du dispositif et un porte-appareil (16) qui porte aussi bien le dispositif photographique (18, 20) que le projecteur (22), le porte-appareil (16) et la tête pouvant être déplacés l'un par rapport à l'autre dans le sens vertical (X) et dans un sens horizontal (Y) qui coïncide sensiblement avec l'axe optique du dispositif photographique, deux palpeurs d'oreilles (100) pouvant respectivement être appliqués sur la zone entre une des oreilles et le crâne et étant munis de marques (114) qui, une fois le palpeur d'oreille appliqué, sont visibles en même temps avec le côté antérieur de la tête, et un dispositif de guidage parallèle (106) pour chaque palpeur d'oreille permettant un déplacement parallèle du palpeur d'oreille concerné dans le sens vertical et dans le sens horizontal précités, les dispositifs de guidage parallèles étant fixés sur le siège (6) ou sur le porte-appareil (16).

2. Dispositif selon la revendication 1, caractérisé en ce que le porte-appareil (16) peut être déplacé dans le sens vertical ( X) et dans l'un des sens horizontaux (Y) par rapport au socle (2) solidaire du dispositif.

3. Dispositif selon la revendication 2, caractérisé en ce que le porte-appareil (16) peut être déplacé dans un second sens horizontal qui s'étend sensiblement perpendiculairement au premier sens horizontal.

4. Dispositif selon l'une des revendications 1 à 3, caractérisé par un chariot qui peut se déplacer sur le socle (2) dans le premier sens horizontal et porte le porte-appareil (16).

5. Dispositif selon la revendication 4, caractérisé par au moins une broche d'entraînement (124) qui, s'étendant sensiblement verticalement, s'appuie sur le chariot et est en prise avec un écrou fixe (126) solidaire du porte-appareil (16), et par un dispositif de guidage (120, 122) pour guider le porte-appareil sur le chariot lors de son mouvement vertical.

6. Dispositif selon la revendication 1, caractérisé par une colonne (132) verticale fixe solidaire du socle (2) et par un chariot (138) pouvant se déplacer verticalement sur le colonne, lequel porte le porte-appareil (16) de telle sorte qu'il peut se déplacer sur le chariot dans le premier sens horizontal.

7. Dispositif selon l'une des revendications 1 à 6, caractérisé par des dispositifs d'entraînement par moteur électrique pour permettre les mouvements du porte-appareil (16) dans les sens de déplacement qui lui sont possibles.

8. Dispositif selon l'une des revendications 1 à 7, caractérisé par un support (38) pour le projecteur (22) dans le porte-appareil (16), lequel support permet un pivotement du projecteur de telle sorte que l'image modèle (44) projetée soit déplacée horizontalement, et par un vérin (50) pour faire pivoter le support.

9. Dispositif selon l'une des revendications 1 à 8, comportant une diapositive placée dans le projecteur, caractérisé en ce que la diapositive (42) dans le projecteur peut tourner autour de l'axe optique de celui-ci et en ce qu'on prévoit un vérin (46) pour faire tourner la diapositive.

10. Dispositif selon l'une des revendications 1 à 9, caractérisé en ce qu'au moyen du dispositif photographique (18, 20) on peut prendre simultanément de plusieurs directions deux clichés de la tête avec l'image modèle projetée sur elle.

11. Dispositif selon la revendication 10, caractérisé en ce que le dispositif photographique comporte un appareil photographique muni d'un dispositif qui produit les images associées aux deux clichés dans deux zones différentes du plan de l'image de l'appareil photographique.

12. Dispositif selon la revendication 10, caractérisé en ce que le dispositif photographique comporte deux appareils photographiques (18, 20).

13. Dispositif selon la revendication 12, caractérisé en ce que l'un des appareils photographiques (20) est placé au dessus du projecteur (22) et l'autre appareil photographique (18) est placé en dessous du projecteur (22).

14. Dispositif selon l'une des revendications 10 à 12, caractérisé en ce que chaque appareil photographique (18, 20) est une caméra vidéo.

15. Dispositif selon la revendication 14, caractérisé en ce que chaque caméra (18, 20) est une caméra vidéo numérique.

16. Dispositif selon l'une des revendications 1 à 15, caractérisé par un objet d'observation (24) porté par le porte-appareil (16) et un agencement de lentilles (26) associé à l'objet d'observation, laquelle fait apparaître l'objet d'observation à grande distance.

17. Dispositif selon l'une des revendications 1 à 16, caractérisé par un dispositif de mesure d'éloignement (88, 89) pour mesurer l'éloignement de la tête par rapport au dispositif de caméras (18, 20) et au projecteur (22).

18. Dispositif selon la revendication 17, caractérisé en ce que le dispositif de mesure d'éloignement comporte deux barrages photographiques munis chacun d'un récepteur optoélectronique (88, 89) et d'un émetteur associé, les deux barrages photoélectriques s'étendant sensiblement horizontalement transversalement à l'axe optique de dispositif de caméras (18, 20).

19. Dispositif selon l'une des revendications 1 à 18, caractérisé par un dispositif d'éclairage (78) disposé sur le porte-appareil (16) pour éclairer la tête sensiblement par devant.

20. Dispositif selon l'une des revendications 1 à 19, caractérisé par un dispositif d'éclairage des orbites oculaires (82, 84, 86) qui envoie deux rayons lumineux d'éclairage orientés en oblique vers le haut.

21. Dispositif selon l'une des revendications 1 à 20, caractérisé par un objet de calibrage (90) qui peut être déplacé dans une position de calibrage et dans une position de repos, l'objet de calibrage prenant dans sa position de calibrage sensiblement la position de la tête pendant la mesure, et étant disposé à l'extérieur du champ visuel du dispositif de caméras (10, 20) dans sa position de repos.

22. Dispositif selon la revendication 21, caractérisé en ce que l'objet de calibrage (90) est monté sur le porte-appareil (16).

23. Dispositif selon l'une des revendications 1 à 22, caractérisé par un capot fixé sur le porte-appareil (16) et comportant deux parois latérales (68, 70), une paroi de fond (72), une paroi supérieure (64) et une paroi frontale (66), la paroi frontale étant munie d'une ouverture (74) pour la tête et étant à une distance du porte-appareil qui est sensiblement celle entre la tête et le porte-appareil pendant la mesure, et le capot obscurcissant vers l'extérieur l'espace entre le dispositif de caméras (18, 20) et le projecteur (22), d'une part et la paroi frontale (66), d'autre part.

24. Dispositif selon la revendication 23, en liaison avec l'une des revendications 17 et 18, ainsi qu'en liaison avec les revendications 20 et 22, caractérisé en ce que le dispositif de mesure d'éloignement (88, 89), le dispositif d'éclairage des orbites oculaires (82, 84, 86), l'objet de calibrage (90) et les deux dispositifs de guidage parallèles (106) des palpeurs d'oreilles (100) sont disposés sur ou dans le capot ( 64, 66, 68, 70, 72).

25. Dispositif selon la revendication 18, caractérisé par au moins un bras (140, 142) fixé sur le porte-appareil (16), qui s'étend jusqu'aux emplacements des deux émetteurs et des deux récepteurs (88, 89) et qui porte les émetteurs et les récepteurs.

26. Dispositif selon les revendications 25 et 20, caractérisé en ce que le(s) bras (140, 142) porte en outre le dispositif d'éclairage des orbites oculaires (86).

27. Dispositif selon la revendication 25 ou 26, caractérisé par un couvercle (146) pouvant être placé de façon amovible sur le(s) bras (140, 142) et le porte-appareil (16), lequel couvercle obscurcit l'espace entre le dispositif de caméras (18, 20) et le projecteur (22), d'une part, et le dispositif de mesure d'éloignement (88, 89) d'autre part.

Fig. 1

Fig. 2

Fig. 3

Fig. 4

Fig. 5

Fig.6

Fig. 7

Fig. 8

Fig. 9

Fig. 10

Fig. 12

Fig. 11